# EUROPEAN PATENT SPECIFICATION

(11) **EP 1 838 862 B1**
(45) Date of publication and mention of the grant of the patent: **11.10.2017**
(21) Application number: 05746129.5
(22) Date of filing: 30.05.2005
(51) Int. Cl.: C12P 17/06, C07D 311/04, C07D 311/30

(54) **MANUFACTURING METHOD OF KAEMPFEROL**
HERSTELLUNGSVERFAHREN FÜR KAEMPFEROL
PROCEDE DE FABRICATION DU KAEMPFEROL

(30) Priority: 18.01.2005 KR 20050004715
(43) Date of publication of application: 03.10.2007
(73) Proprietor: Amorepacific Corporation, Seoul 140-777 (KR)
(72) Inventor: YEOM, Myeong Hoon, 104-1101, Byuksan-Apartment, Yongin-city, Gyonggi-do, 449-162 (KR); PARK, Jun-seong, Yongin-city Gyonggi-do, 449-905 (KR); PARK, Won-seok, Seoul, 135-080 (KR); JOO, Kyungmi, 835-1703, Joogong-Apartment, Soowon-city, Gyonggi-do, 443-725 (KR); RHO, HO Sik, Yongin-city, Gyonggi-do, 449-903 (KR); KIM, Duck Hee, Seoul, 137-030 (KR); JANG, Ih Seop, Yongin-city, Gyonggi-do, 449-170 (KR); LEE, Ok-Sub,2309-1504, LG-Village-Apartment, Yongin-city, Gyonggi-do, 449-140 (KR)
(74) Representative: Novagraaf Technologies
(86) International application number: PCT/KR2005/001597
(87) International publication number: WO 2006/093368

(56) References cited:
- FINGER A ET AL: "Flavonol triglycosides containing galactose in tea" PHYTOCHEMISTRY, PERGAMON PRESS, GB LNKD- DOI:10.1016/0031-9422(91)85066-9, vol. 30, no. 6, 1 January 1991 (1991-01-01), pages 2057-2060, XP026630988 ISSN: 0031-9422 [retrieved on 1991-01-01]
- A. FINGER ET AL.: "Flavonol Glycosides in Tea - Kaempferol and Quercitin Rhamnodiglucosides" J. SCI. FOOD AGRIC., vol. 55, 1991, pages 313-321, XP002596956
- BUDZIANOWSKI ET AL: "Kaempferol glycosides from Hosta ventricosa" PHYTOCHEMISTRY, PERGAMON PRESS, GB LNKD- DOI:10.1016/0031-9422(90)85292-N, vol. 29, no. 11, 1 January 1990 (1990-01-01), pages 3643-3647, XP026615918 ISSN: 0031-9422 [retrieved on 1990-01-01]
- K. NAKANO ET AL.: "Four Kaempferol Glycosides from Leaves of Cinnamomum Sieboldii" PHYTOCHEMISTRY, vol. 22, no. 12, 1983, pages 2831-2833, XP002596957
- F. SOLIMAN ET AL.: "An Acetylated Kaempferol Glycoside from Flowers of Foeniculum vulgare and F. Dulce" MOLECULES, vol. 7, 2002, pages 245-251, XP002596958
- BOKKENHEUSER V.D. ET AL.: 'Hydrolysis of dietary flavonoid glycosides by strains of intestinal Bacterides from humans' BIOCHEM. J. vol. 248, 1987, pages 953 - 956, XP008115182
- SEKINE T. ET AL.: 'Two flavonol glycosides from seeds of Camellia sinensis' PHYTOCHEMISTRY vol. 30, no. 3, 1991, pages 991 - 995, XP026632278
- SCHNEIDER H. ET AL.: 'Anaerobic degradation of flavonoids by Eubacterium ramulus' ARCH. MICROBIOL. vol. 173, no. 1, 2000, pages 71 - 75, XP008115183
- MO Y.Y. ET AL.: 'Analysis of sweet cherry (Prunus avium L.) leaves for plant signal molecules that activate the syrB gene required for synthesis of the phytotoxin, syringomycin, by Pseudomonas syringae pv syringae' PLANT PHYSIOL. vol. 107, no. 2, 1995, pages 603 - 612, XP002270982

## Description

### Technical Field

The present invention relates to a manufacturing method of kaempferol wherein kaempferol is isolated from kaempferol glycosides using an enzyme combination.

### Background Art

Kaempferol having a following chemical formula 1 is one of representative ingredients of flavonol which is one of flavonoids, and widely distributed in a flower or leaf of a plant.

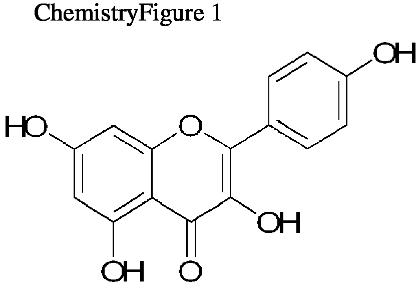

100 or more of types of flavonols have been already known and it is known that kaempferol, quercetin and myricetin among them exist most.

In particular, kaempferol is a substance having excellent physiological activities such as anti-oxidation and anti-inflammatory activities. Accordingly, researches on the various efficacies of kaempferol have been performed and kaempferol was applied to diverse fields. However, since kaempferol, which is currently used, is mostly a plant extract which contains it in an amount of several ppm to several tens ppm only, a substantial efficacy of kaempferol is difficult to be revealed. In addition, since it is difficult to find a plant containing a large quantity of kaempferol and there are no economical merits of isolation and purification for preparing a large quantity of kaempferol, researches on a mass production of kaempferol has seldom been carried out.

Green tea is a beverage having the oldest history in the world. As a concern about the green tea increases in recent years, there have been many researches on ingredients and pharmacological efficacies of the tea. The green tea contains a large amount of threamines and polyphenols, compared to other foods. It is known that a functional ingredient of the green tea is flavan-3-ol-based catechin belonging to multifoliate polyphenols and main ingredients thereof are (+)-catechin, (-)-epicatechin, (-)-epigallocatechin-3-gallate, and (-)-gallocatechin, etc. In particular, it is reported that polyphenols contained in the green tea lowers the level of cholesterol in blood and have anti-oxidization, anti-cancer, detoxification, antibacterial fonction, tooth decay prevention, aging suppression actions, a whitening effect and a fragrance ingredient, etc. It is also reported that polyphenols contained in the green tea prevent gout, suppresses lipid peroxide and a production of neutral lipid and delays the aging, thereby preventing obesity and improving resisting power of a capillary vessel.

However, the green tea having the various efficacies is mostly used in a form of leaf and a green tea seed containing similar effective ingredients is not used besides a cultivation purpose. In addition, ail concerns and researches are focused on catechins of the green tea, especially epigallocatechin gallate (EGCG).

Finger et al. (« Flavonol triglycosides containing galactose in tea », Phytochemistry, Pergamon Press, vol. 30, no. 6, 1 January 1991, p. 2057-2060) discloses the isolation of kaempferol triglycosides (kaempferol glucosylrhamnosylgalactosides) from Camillia sinensis (black tea). Tea was extracted with MeOH (methanol), followed by filtration. The residue was extracted x2 with methanol and then purified. Then, sugars and mineral were removed. Flavonol glycosides were eluted with methanol. The kaempferol glucosylrhamnosylgalactoside was purified by three further steps of HPLC. Then, an acid hydrolysis revealed kaempferol, glucose, rhamnose and galactose as products.

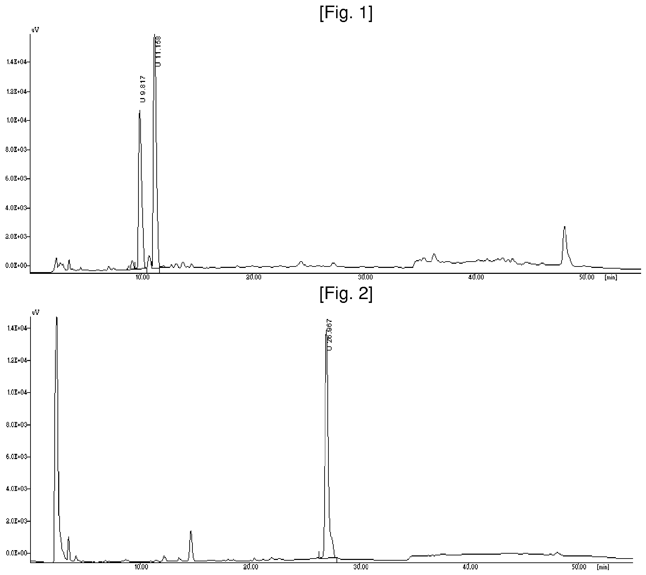

Finger et al. ("Flavonol glycosides in tea-Kaempferol and quercetin Rhamnodiglucosides", J.Sci. Food Agric., vol. 55,1991, p. 313-321) discloses the isolation of kaempferol-3-0-[β-D-glucopyranosyl-(1→3)-α-L-rhamnopyranosyl-(1→6)-β-D-glucopyranosides. Tea was extracted with methanol, followed by filtration and purification by column chromatography. HPLC separation showed a kaempferol derivative (peak 10 on Fig. 1=kaempferol-3-triglycoside). An enzymic hydrolysis was carried out as described by Siewek and Galensa and revealed kaempferol, glucose and rhamnose. The exact structure of the kaempferoldiglucoside is kaempferol-3-O-[β-D-glucopyranosyl-(1→3)-α-L-rhamnopyranosyl-(1→6)-β-D-glucopyranosides.

Budzianowski et al. ("Kaempferol glycosides from Hosta ventricosa", Phytochemistry, Pergamon Press, vol. 29, no. 11, 1 January 1990, p. 3643-3647) discloses that leaves of *Hosta ventricosa* yielded eight kaempferol glycosides: 3-(2G-glucosylrutinoside)-7-glucoside, kaempferol 3-O-sophoroside-7-O-glucoside, kaempferol 3,7-derivative containing glucose, rhamnose and xylose and yielding compounds identical to compound 6 and compound 8 upon β-glucosidase hydrolysis, kaempferol 3-O-rutinoside-7-O-glucoside, kaempferol 3-O-(2G-glucosylrutinoside), kaempferol 3-derivative containing glucose, rhamnose and xylose, kaempferol 3-O-sophoroside and kaempferol 3-rutinoside.

Nakano et al. ("Four Kaempferol glycosides from leaves of Cinnamomum Sieboldii", Phytochemistry, vol. 22, no. 12, 1983, p. 2831-2833) discloses four flavonol glycosides that were isolated from Cinnamomum sielboldii and characterized as: Kaempferol 3,7-O-bis-α-L-rhamnopyranoside, 3-0-α-L-arabinofuranosyl kaempferol 7-O-α-L-rhamnopyranoside, 3-O-β-D-apiofuranosyl-(1→2)-α-L-arabinofuranosyl kaempferol 7-O-α-L-rhamnopyranoside, 3-O-β-D-glucopyranosyl-(1→3)-α-L-rhamnopyranosyl kaempferol 7-O-α-L-rhamnopyranoside.

Soliman et al. ("An acetylated Kaempferol glycoside from flowers of Foeniculum vulgare and F. Dulce", Molecules, vol. 7, 2002, p. 245-251) discloses an acylated kaempferol glycosides, namely kaempferol-3-O-α-L-(2",3"-di-E-p-coumaroyl)-rhamnoside and kaempferol-3-O-α-L-rhamnoside from the flowers of Foeniculum vulgare Mill. and F. dulce DC. The air-dried flowers of F. vulgaris were extracted with ethanol to get dry residue. This residue is then suspended in water and chromatographed to obtain these compounds. Acid hydrolysis of the second compound gave kaempferol and L-rhamnose.

Bokkenheuser et al. ("Hydrolysis of dietary flavonoid glycosides by strains of intestinal Bacterides from humans", Biochem. J., vol. 248, 1987, p. 953-956) discloses that robinin is hydrolysed to kaempferol by faecal flora from healthy subjects, by rhamnosidase and galactosidase that are produced by bacterial strains.

Sekine et al. ("Two flavonol glycosides from needs of Camellia sinensis", Phytochemistry, vol. 30, no. 3, 1991, p. 991-995) discloses the isolation of camelliaside A and camelliaside B from seeds of Camellia sinensis. Camelliaside A and camelliaside B were hydrolyzed with crude hesperidinase or with crude cellulase or with β-glucosidase. The compounds obtained were: from hesperidinase hydrolysis of camelliaside A: kaempferol 3-O-β-galactopyranosyl(1→2)-β-glucopyranoside and kaempferol 3-O-α-L-rhamnopyranosyl(1→6)-β-D-glucopyranoside, or from hesperidinase hydrolysis of camelliaside B: kaempferol 3-O-β-xylopyranosyl(1→2)-β-glucopyranoside, or from cellulase hydrolysis of camelliasides A and B.

### Disclosure of Invention

### Technical Problem

The inventors discovered that a green tea seed, which is not used for a specific purpose, and a leaf of the green tea, which is focused on EGCG, contain a large quantity of kaempferol glycosides having a kaempferol mother nucleus, in particular, glycosides such as camelliaside A and camelliaside B having three sugars attached to kaempferol. From this discovery, the inventors accomplished a method for mass-producing an aglycone type of kaempferol having an excellent physiological activity.

Accordingly, it is described a method of isolating and preparing kaempferol from kaempferol glycosides, using an acid, a base, an enzyme or a microbe producing the enzyme. The object of the invention is to provide a method of isolating and preparing kaempferol from kaempferol glycosides abundantly contained in a green tea seed or leaf, in particular glycosides such as camelliaside A and camelliaside B using an enzyme combination according to claim 1.

### Technical Solution

The object of the present invention is to provide a method for manufacturing kaempferol comprising hydrolyzing kaempferol glycosides using an enzyme combination of cellulase, hesperidinase, naringinase, glucuronidase, galactosidase and amyloglucosidase, wherein the kaempferol glycosides comprise camelliaside A and/or camelliaside B.

The method according to the invention may compris, prior to hydrolyzing kaempferol glycosides, extraction of said kaempferol glycosides from a plant using water or an organic solvent.

In the method according to the invention, the plant may be a seed or leaf of green tea.

In the method according to the invention, the organic solvent may be at least one selected from a group consisting of ethanol, methanol, butanol, ether, ethyl acetate and chloroform, or a mixture solvent of the organic solvent(s) and water.

More specifically, the kaempferol preparing method comprises a first step of obtaining a plant extract containing kaempferol glycosides from a plant, using water or an organic solvent; and a second step of hydrolyzing the plant extract using an enzyme combination.

The kaempferol glycosides comprise camelliaside A or camelliaside B.

According to an embodiment of the invention, in the first step, the plant extract may be derived from a green tea seed or leaf

In addition, according to an embodiment of the invention, the organic solvent may be at least one selected from a group consisting of ethanol, methanol, butanol, ether, ethylacetate and chloroform, or a mixture solvent of the organic solvents and water, preferably 80% ethanol.

The enzyme composition used for hydrolysing kaempferol glycosides is a combination of cellulase, hesperidinase, naringinase, glucuronidase, galactosidase and amyloglucosidase.

Further, it is described that the microbe producing the enzyme may be at least one selected from a group consisting of *aspergillus sp., bacillus sp., penicillium sp., rhizopus sp., rhizomucor sp., talaromyces sp., bifidobacterium sp., mortierella sp., cryptococcus sp.* and *microbacterium sp.*

### Advantageous Effects

When using the method of isolating kaempferol from kaempferol glycosides with the enzyme composition, it is possible to mass-produce kaempferol, which is one of main physiological active ingredients.

### Brief Description of the Drawings

FIGS. 1 and 2 show results of high speed liquid chromatography measurement measuring changes before and after hydrolyzing using an enzyme an extract of green tea seed of the Example 1 according to a method of the Example 3, wherein FIG. 1 is a view showing contents of camelliaside A and camelliaside B in the extract of green tea before the hydrolysis and FIG. 2 is a view showing a content of kaempferol in the extract of green tea after the hydrolysis.

### Best Mode for Carrying Out the Invention

The kaempferol preparing method according to the invention comprises a first step of obtaining a plant extract containing kaempferol glycosides from a plant using water or an organic solvent; and a second step of hydrolyzing the plant extract using an enzyme composition according to claim 1.

In the first step, in order to obtain the plant extract containing camelliaside A or camelliaside B, which are kaempferol glycosides, using the water or organic solvent, about one or six times, preferably about three times water or at least one organic solvent selected from a group consisting of ethanol, methanol, butanol, ether, ethylacetate and chloroform, or a mixture solvent of the organic solvents and water is poured to the plant. Then, the plant is extracted while being stirred one to five times at a room temperature to remove fat. About one to eight times, preferably about four times water or the organic solvent is poured to the fat-removed plant. The mixture is extracted under reflux one to five times, and deposited at 10 to 20°C for one to three days. After that, residues and filtrate are separated through filtration and centrifugation, and extracts obtained by concentrating under reduced pressure the separated filtrate are suspended in water and pigments thereof are removed using ether, etc. Then, water layer is removed one to five times using butanol, etc. After that, an extract is obtained by concentrating under reduced pressure the obtained organic solvent layer, and dissolved in a small quantity of methanol, etc. Then, precipitates produced by adding a large quantity of ethylacetate, etc. to the mixture are dried to obtain the plant extract of the invention.

In the second step, the plant extract is hydrolyzed using an enzyme composition of claim 1 to prepare kaempferol.

When the enzyme composition of claim 1 is used, the plant extract is dissolved in 5 to 20 times, preferably about 10 times acid buffer solution, added with the enzymes and then stirred in a water bath at about 37°C for 40 to 55 hours, preferably 48 hours. At the same time, a removal rate of substrate is checked with a thin layer chromatography. When the substrate is completely removed, the mixture is heated in a hot water (80∼100°C) for 5 to 15 minutes to terminate the hydrolysis reaction, thereby providing a reaction solution.

As described above, the hydrolysis reaction is performed using an enzyme combination as described in claim 1. Then, the reaction solution obtained is concentrated under reflux pressure to remove the solvent and alcohol is added to the residues and then the mixture is stirred one to five times. Precipitated salts are removed through a filtration, and filtered filtrate is concentrated under reduced pressure to obtain crude products. The obtained crude products are purified with a silica gel column chromatography (chloroform: methanol = 8:1 ∼ 4:1), thereby providing kaempferol.

### Mode for the Invention

Hereinafter, the invention will be more specifically described with examples and experimental examples.

### Example 1: Preparation of extract of a green tea seed

61 of hexane was added to 2 kg of green tea seeds and then the mixture was stirred three times at a room temperature to remove fat. 41 of 80% methanol was poured to 1kg of the fat-removed seeds, the mixture was extracted under reflux three times and then deposited at 15°C for a day. After that, residues and filtrate were separated through filtration of filter cloth and centrifugation and an extract obtained by concentrating under reduced pressure the separated filtrate was suspended in water and then extracted five times with 11 of ether to remove pigments. Water layer was extracted three times with 500 **ml** 1-butanol. All 1-butanol layer obtained was concentrated under reduced pressure to obtain 1-butanol extract. The obtained extract was dissolved in a small quantity of methanol and then added to a large quantity of ethylacetate, thereby obtaining precipitates. The produced precipitates were dried, thereby obtaining 250g of extract of green tea seeds.

### Example 2: Preparation of extract of a green tea leaf

61 of hexane was added to 2 kg of green tea leaves and then the mixture was stirred three times at a room temperature to remove fat. 41 of 80% methanol was poured to 1 kg of the fat-removed leaves, the mixture was extracted under reflux three times and then precipitated at 15°C for a day. After that, residues and filtrate were separated through filtration of filter cloth and centrifugation and an extract obtained by con- centrating under reduced pressure the separated filtrate was suspended in water and then extracted five times with 11 of ether to remove pigments. Water layer was extracted three times with 500 **ml** 1-butanol. All 1-butanol layer obtained was con- centrated under reduced pressure to obtain 1-butanol extract. The obtained extract was dissolved in a small amount of methanol and then added to a large quantity of ethylacetate. The produced precipitates were dried, thereby obtaining 150g of extract of green tea leaves.

### Reference Example 3: Preparation of kaempferol with an acid hydrolysis method

10 g of the extract of green tea seeds, which was obtained from the Example 1, was added to 20 times (v/w) 1N HCl-50% methanol solution (v/v) and then subject to a reflux-heating in a water bath at 80°C for 8 hours, thereby hydrolyzing sugars bonded to camelliaside A and camelliaside B. After that, the reaction solution was con- centrated under reduced pressure to remove the solvent, and ethanol (200 **ml**) was added to the residues and the mixture was stirred (three times). The resulting precipitated salts were removed through filtration, and filtered filtrate was concentrated under reduced pressure to obtain crude products. The obtained crude products were purified with a silica gel column chromatography (chloroform: methanol = 8:1 ∼ 4:1), thereby obtaining 0.95 g of kaempferol.

### Reference Example 4: Preparation of kaempferol with a base hydrolysis method

10 g of the extract of green tea seeds, which was obtained from the Example 1, was dissolved in dry pyridine (500 **ml**), added to sodium methoxide (powder, lüg) and was then subject to a reflux-heating in a water bath for 8 hours, thereby hydrolyzing sugars bonded to camelliaside A and camelliaside B. After that, the reaction solution was con- centrated under reduced pressure to remove the solvent, and ethanol (200 **ml**) was added to the residues and the mixture was stirred (three times). The resulting precipitated salts were removed through filtration. Filtered filtrate was concentrated under reduced pressure to obtain crude products. The obtained crude products were purified with a silica gel column chromatography (chloroform: methanol = 8:1 ∼ 4:1), thereby obtaining 0.25 g of kaempferol.

### Example 5: Preparation of kaempferol with an enzyme digestion method

10 g of the extract of green tea seeds, which was obtained from the example 1, was dissolved in 100 **ml** of 0.1 M acetic acid buffer solution (pH 4.5). 2.5 g of enzyme (hesperidinase 0.5g, naringinase 0.5g, cellulase 0.5g, β-glucuronidase 0.2g, β-galactosidase 0.5g, amyloglucosidase 0.3g; manufactured from Sigma company) was added to the mixture and the mixture was periodically checked with a thin layer chromatography while being stirred in a water bath at 37°C for 48 hours. When the substrate (camelliaside A and camelliaside B) was completely removed, the mixture was heated in hot water (80-100°C) for 10 minutes to terminate the reaction. After that, the reaction solution was concentrated under reduced pressure to remove the solvent, and the residues were added to ethanol (200 **ml**) and stirred (three times). The resulting precipitates were removed through filtration and filtered filtrate was then concentrated under reduced pressure to obtain crude products. The obtained crude products were purified with a silica gel column chromatography (chloroform: methanol = 8:1 ∼ 4:1), thereby obtaining 1.02 g of kaempferol.

### Reference Example 6: Preparation of kaempferol using microbes

10 g of the extract of green tea leaves, which was obtained from the Example 2, was dissolved in 100 **ml** of ionic water, sterilized at 121 °C for 30 minutes, cooled to 30°C, inoculated with 5∼10%, based on a liquid amount, *Aspergillus niger* KCCM 11885 cultured in advance and cultivated at 30°C for 5 days. A removal rate of substrate was checked with a thin layer chromatography. When the substrate was completely removed, the hydrolysis reaction was terminated and precipitates recovered by centrifuging the culture solution at 5,000 to 10,000 rpm were cleaned three times with distilled water and then centrifuged, thereby obtaining a reaction solution as precipitates. ethanol (200 **ml**) was added to the precipitates and then the mixture was stirred (three times). Then, the precipitates were removed through filtration and filtered filtrate was concentrated under reduced pressure to obtain crude products. The obtained crude products were purified with a silica gel column chromatography (chloroform: methanol = 8:1∼4:1), thereby obtaining 0.62 g ofkaempferol.

### Experimental example 1: Identification of camelliaside A and camelliaside B

10 g of the extract of green tea seeds, which was obtained from the Example 1, was purified with a silica gel column chromatography (filled with 100 g silica gel). At this time, chloroform and methanol were used as a development solvent and a ratio of chloroform and methanol was increased from 10: 1 to 2: 1 so as to raise a concentration gradient and thus to obtain a fractionation. From the fractionation, 0.82g camelliaside A and 1.24g camelliaside B were obtained. The obtained products were subject to an identification process (Varian Gemini 2000, 300 MHz, Varian company). As a result of that, since the products exhibited characteristics as shown in the following Table 1, they were identified as camelliaside A and camelliaside B.

### <physicochemical properties of camelliaside A>

property: light greenish yellow micro crystal
positive FAB-MS: 756.9[M+H]

### <physicochemical properties of camelliaside B>

property: light greenish yellow micro crystal
positive FAB-MS: 726.9[M+H]

**Table 1 ¹H-NMR and ¹³C-NMR data of camelliaside A and camelliaside B**

| Camelliaside A | | | Camelliaside B | | |
|---|---|---|---|---|---|
| ¹³C | | ¹H | ¹³C | | ¹H |
| kaempferol | | 1.09(3H,d, 6.3 Hz) | kaempferol | | 1.09(3H,d, 6.3 Hz) |
| 2 | 159.247 | 3.2-3.8(16H, m) | 2 | 158.697 | 3.2-3.8(15H, m) |
| 3 | 134.717 | 4.4(1H,d,H1-Rha) | 3 | 134.827 | 4.4(1H,d,H1-Rha) |
| 4 | 179.475 | 4.7(1H,d,H1-Gal) | 4 | 179.425 | 4.7(1H,d,H1-Gal) |
| 5 | 161.467 | 5.3(1H,d,7.8Hz,H1-G1 c) | 5 | 161.363 | 5.3(1H,d,7.8Hz,H1-G1 c) |
| 6 | 101.122 | 6.17(1H,d,1.8Hz,H6) | 6 | 99.919 | 6.17(1H,d,1.8Hz,H6) |
| 7 | 163.019 | 6.37(1H,d,1.8Hz,H8) | 7 | 163.038 | 6.37(1H,d,1.8Hz,H8) |
| 8 | 95.063 | 6.9(2H,d,9Hz,H3',5') | 8 | 94.847 | 6.9(2H,d,9Hz,H3',5') |
| 9 | 166.589 | 8.0(2H,d,8.7Hz,H2',6' ) | 9 | 165.906 | 8.0(2H,d,8.7Hz,H2',6) |
| 10 | 105.565 | | 10 | 105.725 | |
| 1' | 122.936 | | 1' | 122.924 | |
| 2', 6' | 132.365 | | 2', 6' | 132.346 | |
| 3', 5' | 116.239 | | 3', 5' | 116.167 | |
| 4' | 158.595 | | 4' | 158.473 | |
| Glc | | | Glc | | |
| 1 | 101.122 | | 1 | 100.830 | |
| 2 | 82.048 | | 2 | 81.927 | |
| 3 | 78.281 | | 3 | 78.205 | |
| 4 | 72.073 | | 4 | 71.424 | |
| 5 | 77.818 | | 5 | 76.839 | |
| 6 | 68.226 | | 6 | 68.112 | |
| Rha | | | Rha | | |
| 1 | 102.211 | | 1 | 102.139 | |
| 2 | 72.293 | | 2 | 72.274 | |
| 3 | 73.856 | | 3 | 73.853 | |
| 4 | 71.402 | | 4 | 72.062 | |
| 5 | 69.713 | | 5 | 69.698 | |
| 6 | 17.853 | | 6 | 17.845 | |
| Gal | | | Xyl | | |
| 1 | 104.476 | | 1 | 105.133 | |
| 2 | 75.378 | | 2 | 74.676 | |
| 3 | 76.945 | | 3 | 77.051 | |
| 4 | 71.274 | | 4 | 70.996 | |
| 5 | 77.867 | | 5 | 66.541 | |
| 6 | 62.591 | | | | |
| *Glc: glucose, Rha: rhamnose, Gal:galactose, Xyl: xylose | | | | | |

### Experimental example 2: Identification of kaempferol

Since the products, which were prepared in the Examples 3 to 6, exhibited characteristics as follows, they were identified as kaempferol (Verlan Gemini 2000, 300 MHz, Varian company)

### <physicochemical properties of kaempferol>

property: light greenish yellow micro crystal
positive FAB-MS: 287[M+H]+
¹H-NMR: 6.1(1H, d, 1.8 Hz), 6.3(1H, d, 1.8 Hz), 6.8(2H, dd, 9Hz), 8.0(2H, dd, 9Hz)
¹³C-NMR: 94.467, 99.248, 104.518, 116.265, 123.710, 130.649, 137.069, 147.970, 158.200, 160.480, 162.446, 165.519, 177.285

### Experimental example 3: Changes of contents of kaempferol after the hydrolysis (enzyme digestion)

The extract of green tea seeds was prepared according to the Example 1 and then subject to the enzyme digestion reaction as described in the Example 3. After that, a change before and after the enzyme digestion reaction was measured using a highspeed liquid chromatography. At this time, measurement results of contents of camelliaside A and camelliaside B, which were contained in the extract of green tea seeds before the enzyme digestion reaction, were shown in Fig. 1, and measurement results of contents of kaempferol after the enzyme digestion reaction were shown in Fig. 2.

As shown in Figs. 1 and 2, almost all of camelliaside A and camelliaside B were converted into kaempferol.

### Industrial Applicability

According to the invention kaempferol is isolated from kaempferol glycosides with an enzyme combination. The plant extracts, particularly a seed or leaf of green tea, contain kaempferol glycosides, particularly camelliaside A or camelliaside B. Thereby it is possible to mass-produce kaempferol, which is one of main physiological active ingredient.

## Claims

1. A method for manufacturing kaempferol comprising hydrolyzing kaempferol glycosides using an enzyme combination of cellulase, hesperidinase, naringinase, glucuronidase, galactosidase and amyloglucosidase, wherein the kaempferol glycosides comprise camelliaside A and/or camelliaside B.

2. The method according to claim 1, which comprises prior to hydrolyzing kaempferol glycosides, extraction of said kaempferol glycosides from a plant using water or an organic solvent.

3. The method according to claim 2, wherein the plant is a seed or leaf of green tea.

4. The method according to claim 2, wherein the organic solvent is at least one selected from a group consisting of ethanol, methanol, butanol, ether, ethyl acetate and chloroform, or a mixture solvent of the organic solvent(s) and water.

## Patentansprüche

1. Verfahren zur Herstellung von Kaempferol, umfassend das Hydrolisieren von Kaempferol-Glykosiden unter Verwendung einer Enzymkombination aus Cellulase, Hesperidinase, Naringinase, Glucuronidase, Galactosidase und Amyloglucosidase. wobei die Kaempferol-Glykoside Camelliasid A oder Camelliasid B umfassen.

2. Verfahren nach Anspruch 1, das vor dem Hydrolisieren der Kaempferol-Glykoside die Extraktion der Kaempferol-Glykoside aus einer Pflanze unter Verwendung von Wasser oder einem organischen Lösungsmittel umfasst.

3. Verfahren nach Anspruch 2, wobei die Pflanze ein Samen oder Blatt von grünem Tee ist.

4. Verfahren nach Anspruch 2, wobei das organische Lösungsmittel mindestens eines ist ausgewählt aus der Gruppe bestehend aus Ethanol, Methanol, Butanol, Ether, Ethylacetat und Chloroform, oder ein Gemisch aus dem/den organischen Lösungsmittel(n) und Wasser.

## Revendications

1. Procédé de fabrication de kaempferol comprenant une hydrolyse de glycosides du kaempferol en utilisant une combinaison d'enzymes de cellulase, hesperidinase, naringinase, glucuronidase, galactosidase et amyloglucosidase, dans lequel les glycosides du kaempferol comprenant la camelliaside A et/ou la camelliaside B.

2. Procédé selon la revendication 1 qui comprend, avant l'hydrolyse des glycosides du kaempferol, une extraction desdits glycosides du kaempferol à partir d'une plante en utilisant de l'eau ou un solvant organique.

3. Procédé selon la revendication 2, dans lequel la plante est une graine ou une feuille de thé vert.

4. Procédé selon la revendication 2, dans lequel le solvant organique est l'un au moins choisi dans le groupe constitué de l'éthanol, du méthanol, du butanol, de l'éther, de l'acétate d'éthyle, et du chloroforme, ou un mélange du ou des solvant(s) organique(s) et d'eau.
